# EUROPÄISCHE PATENTSCHRIFT

(11) **EP 0 852 237 B1**
(45) Veröffentlichungstag und Bekanntmachung des Hinweises auf die Patenterteilung: **19.06.2002**
(21) Anmeldenummer: 97120234.6
(22) Anmeldetag: 19.11.1997
(51) Int. Cl.: C08F 220/04, C08F 246/00, C08F 228/00, A61K 31/795, A61L 27/00, A61L 17/00, A61L 31/00

(54) **Polymere Materialien mit zellproliferationsbegünstigenden Eigenschaften**
Polymers with cell proliferation fevoring properties
Polymères avec des propriétés améliorant la prolifération cellulaire

(30) Priorität: 03.01.1997 DE 19700078
(43) Veröffentlichungstag der Anmeldung: 08.07.1998
(73) Patentinhaber: THERAPEUTIQUES SUBSTITUTIVES Groupement d'Intéret Public, 93430 Villetaneuse (FR)
(72) Erfinder: Hill, Frank, Dr., 40822 Mettmann (DE); Ottersbach, Peter, Dr., 51570 Windeck (DE); Graciella, Pavon-Djavid, 92800 Puteaux (FR); Jozefowicz, Marcel, Prof., 60260 Lamorlaye (FR); Migonney, Veronique, Dr., 95600 Eaubonne (FR); Vairon, Jean-Pierre, Prof., 92340 Bonzy la Reine (FR)
(74) Vertreter: Orès, Bernard

(56) Entgegenhaltungen:
- EP-A- 0 093 489
- EP-A- 0 604 369
- WO-A-90/02145
- WO-A-96/39821
- US-A- 5 278 200

## Beschreibung

Die Erfindung betrifft wasserunlösliche, die Zellproliferation begünstigende Polymere sowie ein Verfahren zu deren Herstellung.

Außerdem betrifft die Erfindung die Verwendung der wasserunlöslichen, die Zellproliferation begünstigenden Polymeren zur Herstellung von Erzeugnissen mit einer die Zellproliferation begünstigenden Oberfläche sowie zur Herstellung von Erzeugnissen mit einer die Zellproliferation begünstigenden Beschichtung aus dem Polymer.

Desweiteren betrifft die Erfindung Erzeugnisse mit einer die Zellproliferation begünstigenden Oberfläche und Erzeugnisse mit einer die Zellproliferation begünstigenden Beschichtung aus dem Polymer.

Bei zahlreichen medizinischen Anwendungen von Materialien wie Polymeren, Keramiken und Metallen, z. B. als Nahtmaterialien, Stents, Implantaten oder Prothesen, müssen gute Verträglichkeiten mit dem Immun- und Komplementsystem sowie dem Blut gewährleistet sein.

Diese oft als Biokompatiblität bezeichnete Eigenschaft schließt die Vermeidung von Abbauerscheinungen der Materialien durch physiologische Komponenten wie Enzyme und Makrophagen ein.

Eine verbesserte Biokompatibilität von medizinisch eingesetzten Ersatzmaterialien läßt sich grundsätzlich durch die Besiedlung mit menschlichen Zellen erreichen. Das in EP 0 290 642 beschriebene Verfahren erfordert zunächst die kovalente Anbindung einer Zwischenschicht aus sogenannten Biopolymeren auf durch Carboxyl-, Amino- und Hydroxylgruppen funktionalisierten Polymeroberflächen. Die erstrebte Biokompatibilität des Materials wird anschließend durch eine sorgfältige, extrakorporale Endothelzellen-Besiedlung der Zwischenschicht erreicht.

WO 90/02145 beschreibt mit dem gleichen Ziel ein Verfahren, bei dem auf fluorhaltige Polymersubstrate durch Bestrahlung mit einer ⁶⁰Co-Quelle oder einem Laser Acrylsäure aufgepfropft wird. Nach einer Reihe von chemischen Oberflächenprozessen erfolgt die gezielte Sorption von Proteinen, der zur Einstellung der Biokompatibilität die Endothelzellen-Besiedlung folgt.

Diese Verfahren sind außerordentlich zeit- und kostenaufwendig und bedürfen bei einer medizinischen Applikation größter Sorgfalt, um die extrakorporal aufgebrachten Endothelzellenschichten nicht zu verletzen. Es ist weiterhin nicht möglich, die Zellbesiedlung und das Zellwachstum (Zellproliferation) in vivo durch den Körper selbst durchführen zu lassen, da vor der Zellbesiedlung eine unerwünschte thrombische Reaktion einsetzt.

Die nachträgliche chemische Modifikation von Oberflächen polymerer Materialien mit der bekannten RGD-Sequenz-Methode (Arginin-Glycin-Asparaginsäure) ist meist nicht gleichmäßig und/oder einheitlich. Es bleiben häufig nicht behandelte Flächen zurück, die als Ausgangspunkte für eine Zellbesiedlung der Oberfläche nicht mehr zur Verfügung stehen (G. Müller, Angewandte Chemie, 104 (1992) 341 ff.).

Aus einem anderen technischen Gebiet sind gemäß US-PS 5 278 200 Polymere bekannt, die Carboxylat- und Sulfonatgruppen in einem dem natürlichen Heparin vergleichbaren Verhältnis enthalten. Diese Polymere weisen antikoagulierende Eigenschaften gegenüber Thrombozyten im Blut auf.

Der vorliegenden Erfindung liegt daher die Aufgabe zugrunde, die Zellproliferation auf Oberflächen von Polymeren zu verbessern.

Es wurde nun überraschenderweise gefunden, daß wasserunlösliche, Carboxylat- und Sulfonatgruppen enthaltende Polymere, erhältlich durch radikalische Copolymerisation von
- einem oder mehreren Carboxylatgruppen-haltigen, aliphatisch ungesättigten Monomeren oder den entsprechend funktionalisierten Derivaten der Monomeren als Komponente I mit
- einem oder mehreren Sulfonatgruppen-haltigen, aliphatisch ungesättigten Monomeren oder den entsprechend funktionalisierten Derivaten der Monomeren als Komponente II und
- einer Komponente III, die ein aliphatisch ungesättigtes Monomer oder mehrere aliphatisch ungesättigte Monomere enthält,
wobei die entsprechend funktionalisierten Derivate nach der Copolymerisation in Carboxylat- und Sulfonatgruppen überführt werden, in der Lage sind, die Zellproliferation zu begünstigen und wobei das Verhältnis der im Polymer enthaltenden Carboxylatgruppen zu Sulfonatgruppen 3 bis 10 ist.

Die Adhäsion und das Wachstum von Zellen wird somit auf den erfindungsgemäßen Polymeren auf physiologisch verträgliche Weise verbessert.

Die erfindungsgemäße Polymere eignen sich somit besonders zur Herstellung von Implantaten, bei denen ein Aufwachsen von körpereigenen oder undifferenzierten Zellen erwünscht ist.

Gegenstand der vorliegenden Erfindung sind daher wasserunlösliche, die Zellproliferation begünstigende, Carboxylat- und Sulfonatgruppen enthaltende Polymere, erhältlich durch radikalische Copolymerisation von
- einem oder mehreren Carboxylatgruppen-haltigen, aliphatisch ungesättigten Monomeren oder den entsprechend funktionalisierten Derivaten der Monomeren als Komponente I mit
- einem oder mehreren Sulfonatgruppen-haltigen, aliphatisch ungesättigten Monomeren oder den entsprechend funktionalisierten Derivaten der Monomeren als Komponente II und
- einer Komponente III, die ein aliphatisch ungesättigtes Monomer oder mehrere aliphatisch ungesättigte Monomere enthält,
wobei die entsprechend funktionalisierten Derivate nach der Copolymerisation in Carboxylat- und Sulfonatgruppen überführt werden und wobei das Verhältnis der im Polymer enthaltenden Carboxylatgruppen zu Sulfonatgruppen 3 bis 10 ist.

Des weiteren ist Gegenstand der vorliegenden Erfindung ein Verfahren zur Herstellung von wasserunlöslichen, die Zellproliferation begünstigenden, Carboxylat- und Sulfonatgruppen enthaltenden Polymeren gemäß den Ansprüchen 6 bis 10, das dadurch gekennzeichnet ist, daß die Polymere durch radikalische Copolymerisation von
- einem oder mehreren Carboxylatgruppen-haltigen, aliphatisch ungesättigten Monomeren oder den entsprechend funktionalisierten Derivaten der Monomeren als Komponente I mit
- einem oder mehreren Sulfonatgruppen-haltigen, aliphatisch ungesättigten Monomeren oder den entsprechend funktionalisierten Derivaten der Monomeren als Komponente II und
- einer Komponente III, die ein aliphatisch ungesättigtes Monomer oder mehrere aliphatisch ungesättigte Monomere enthält,
erhalten werden, wobei die entsprechend funktionalisierten Derivate nach der Copolymerisation in Carboxylat- und Sulfonatgruppen überführt werden und wobei das Verhältnis der im Polymer enthaltenden Carboxylatgruppen zu Sulfonatgruppen 3 bis 10 ist.

Darüber hinaus ist Gegenstand der vorliegenden Erfindung die Verwendung der wasserunlöslichen, die Zellproliferation begünstigenden, Carboxylat- und Sulfonatgruppen enthaltenden Polymeren gemäß den Ansprüchen 1 bis 8 zur Herstellung von Erzeugnissen mit einer die Zellproliferation begünstigenden Oberfläche sowie die Verwendung der wasserunlöslichen, die Zellproliferation begünstigenden, Carboxylat- und Sulfonatgruppen enthaltenden Polymeren gemäß den Ansprüchen 6 bis 10 zur Herstellung von Erzeugnissen mit einer die Zellproliferation begünstigenden Beschichtung aus dem Polymer.

Außerdem ist Gegenstand der vorliegenden Erfindung die Verwendung der wasserunlöslichen, die Zellproliferation begünstigenden, Carboxylat- und Sulfonatgruppen enthaltenden Polymeren gemäß den Ansprüchen 6 bis 10 zur Herstellung von medizintechnischen Artikeln, insbesondere von künstlichen Blutgefäßen mit einer die Zellproliferation begünstigenden Oberfläche.

Des weiteren ist Gegenstand der vorliegenden Erfindung die Verwendung der wasserunlöslichen, die Zellproliferation begünstigenden, Carboxylat- und Sulfonatgruppen enthaltenden Polymeren gemäß den Ansprüchen 6 bis 10 zur Herstellung von medizintechnischen Artikeln aus Kunststoffen, Keramiken oder Metallen mit einer Zellproliferation begünstigenden Beschichtung aus dem Polymer.

Außerdem ist Gegenstand der vorliegenden Erfindung die Verwendung der wasserunlöslichen, die Zellproliferation begünstigenden, Carboxylat- und Sulfonatgruppen enthaltenden Polymeren gemäß den Ansprüchen 6 bis 10 zur Herstellung von künstlichen Blutgefäßen mit einer die Zellproliferation begünstigenden Beschichtung aus dem Polymer.

Ferner sind Gegenstand der vorliegenden Erfindung Erzeugnisse mit einer die Zellproliferation begünstigenden Oberfläche aus wasserunlöslichen, die Zellproliferation begünstigenden, Carboxylat- und Sulfonatgruppen enthaltenden Polymeren gemäß den Ansprüchen 6 bis 10.

Bevorzugt sind solche erfindungsgemäßen Erzeugnisse medizintechnische Artikel, besonders bevorzugt künstliche Blutgefäße.

Außerdem sind Gegenstand der vorliegenden Erfindung Erzeugnisse mit einer die Zellproliferation begünstigenden Beschichtung aus wasserunlöslichen, die Zellproliferation begünstigenden, Carboxylat- und Sulfonatgruppen enthaltenden Polymeren gemäß den Ansprüchen 6 bis 10.

Erzeugnisse mit einer die Zellproliferation begünstigenden Beschichtung aus dem erfindungsgemäßen Polymer sind bevorzugt medizintechnische Artikel, besonders bevorzugt medizintechnische Artikel aus Kunststoffen, Keramiken oder Metallen. Medizintechnische Artikel mit einer die Zellproliferation begünstigenden Beschichtung aus dem erfindungsgemäßen Polymer sind bevorzugt künstliche Blutgefäße.

Die Herstellung der erfindungsgemäßen Polymeren erfolgt durch Copolymerisation von drei Komponenten.

Für die erfindungsgemäßen Polymeren sowie für das erfindungsgemäße Verfahren können als Komponente I auch ein Carboxylat- und Sulfonatgruppen-haltiges, aliphatisch ungesättigtes Monomeres oder mehrere Carboxylat- und Sulfonatgruppen-haltige, aliphatisch ungesättigte Monomeren oder die entsprechend fanktionalisierten Derivate der Monomeren eingesetzt werden.

Weiterhin können für die erfindungsgemäßen Polymeren sowie für das erfindungsgemäße Verfahren als Komponente II auch ein Carboxylat- und Sulfonatgruppen-haltiges, aliphatisch ungesättigtes Monomeres oder mehrere Carboxylat- und Sulfonatgruppenhaltige, aliphatisch ungesättigte Monomeren oder die entsprechend funktionalisierten Derivate der Monomeren eingesetzt werden.

In diesen besonderen Ausführungsform der vorliegenden Erfindung kann für die erfindungsgemäßen Polymeren sowie für das erfindungsgemäße Verfahren Komponente I identisch mit Komponente II sein.

Die für die erfindungsgemäßen Polymeren einzusetzenden aliphatisch ungesättigten Monomeren können sowohl Doppelbindungen als auch Dreifachbindungen enthalten. Vorzugsweise besitzen die Monomeren eine oder zwei Doppelbindungen.

Zur Einbringung von Carboxylatgruppen in die erfindungsgemäßen Polymere eignen sich beispielsweise als Komponente I alle Carboxylatgruppen tragenden polymerisierbaren Verbindungen der Formel 1) oder Mischungen derselben wie z. B.:
1): (CₙH_{2n-q-x}) (COORₖ)ₓ;
   mit
Rₖ = -(CH₂-CH₂-O)_{d}-H, -(CH₂-CH(CH₃)-O)_{d}-H
   oder
Rₖ = -(CH₂-CH₂-CH₂-O)_{d}-H, -(CH₂)_{d}-NH₃₋ₑ (Rₘ)ₑ,
   wobei
Rₘ = -CH₃,-C₂H₅,
d = 0, 1, 2, 3 oder 4,
e = 0, 1, 2 oder 3,
n = 2, 3, 4, 5 oder 6,
q = 0 oder 2
   **und**
**x = 1 oder 2**
bedeuten.

Die Estergruppen werden nach der Polymerisation verseift und liegen somit ionisch vor. Die aliphatisch ungesättigten Monomeren können sowohl geradkettig als auch verzweigt sein.

Auch vom Benzol ableitbare Monomerkomponenten der Summenformel

(C₆H_{6-a-b-c})Aₐ B_{b}(OH)_{c}

sind zur Herstellung der erfindungsgemäßen Polymeren, beispielsweise als Komponente I, geeignet, worin
A = (Cₙ H_{2n-q-x-1}) (COORₖ)ₓ
   mit
Rₖ =-(CH₂-CH₂-O)_{d}-H, -(CH₂-CH(CH₃)-O)_{d}-H
   oder
Rₖ = -(CH₂-CH₂-CH₂-O)_{d}-H, -(CH₂)_{d}-NH₃₋ₑ (Rₘ)ₑ,
   wobei
Rₘ = -CH₃, -C₂H₅
a = 0, 1, 2 oder 3,
b = 0, 1, 2 oder 3,
c = 0, 1, 2 oder 3,
d = 0, 1, 2, 3 oder 4,
e = 0, 1, 2 oder 3,
n = 2, 3, 4, 5 oder 6,
q = 0 oder 2,
x = 0, 1 oder 2
   und
B = -COOH, -SO₃H, -NH₂, -N⁺(CH₃)₃, -O-PO₃H⁻, -OSO₃H
   oder
B =-O-PO⁻₂-O-CH₂-CH₂-N⁺(CH₃)₃;
bedeuten.

Die Einbringung von Sulfonat-Gruppen in die erfindungsgemäßen Polymeren kann mit folgenden Verbindungen der Formel 2) oder Mischung, n derselben als Komponente II erfolgen:
2): (CₘH_{2m-s-y}) (SO₃Rₗ)_{y}
   mit
Rₗ =-(CH₂-CH₂-O)_{d}-H, -(CH₂-CH(CH₃)-O)_{d}-H,
   oder
Rₗ = -(CH₂-CH₂-CH₂-O)_{d}-H, -(CH₂) -NH₃₋ₑ (Rₘ)ₑ,
   wobei
Rₘ=-CH₃,-C₂H₅,
d = 0, 1, 2, 3 oder 4,
e = 0, 1, 2 oder 3,
m = 0, 2, 3, 4, 5 oder 6,
s = 0 oder 2
   und
y = 1 oder 2
bedeuten.

Die Estergruppen werden nach der Polymerisation verseift und liegen somit ionisch vor. Die aliphatisch ungesättigten Monomeren können sowohl geradkettig als auch verzweigt sein.

Auch vom Benzol ableitbare Monomerkomponenten der Summenformel

(C₆H₆₋ₖ₋ᵢ₋ₚ)KₖLᵢ(OH)ₚ

können zur Herstellung der erfindungsgemäßen Polymeren, beispielsweise als Komponente II, eingesetzt werden, worin
K = (Cₘ H_{2m-s-y-1})(SO₃Rₗ)_{y},
   wobei
d = 0, 1, 2, 3 oder 4,
e = 0, 1, 2 oder 3,
i = 0, 1, 2 oder 3,
k = 0, 1, 2 oder 3,
m = 0, 2, 3, 4, 5 oder 6,
p = 0, 1, 2 oder 3,
s = 0 oder 2,
y = 0, 1 oder 2
   und
L = -COOH, -SO₃H, -NH₂, -N⁺(CH₃)₃, -O-PO₃H⁻, -OSO₃H
   oder
L = -O-PO⁻₂-O-CH₂-CH₂-N⁺(CH₃)₃
bedeuten.

Vorzugsweise beträgt für die erfindungsgemäßen Polymeren sowie für das erfindungsgemäße Verfahren die Summe der Anteile von Komponente I und Komponente II bis 30 Mol-%, besonders bevorzugt 15 bis 20 Mol-%.

Für die erfindungsgemäßen Polymeren sowie für das erfindungsgemäße Verfahren beträgt das Verhältnis der im Polymer enthaltenden Carboxylatgruppen zu Sulfonatgruppen 3 bis 10, besonders bevorzugt 3 bis 5.

Die Copolymerisation der oben genannten Monomeren als Komponenten I und II wird erfindungsgemäß mit einem oder mehreren weiteren aliphatisch ungesättigten Monomeren als Komponente III durchgeführt.

Vorzugsweise wird als Komponente III ein nicht ionische Gruppen tragendes Monomer verwendet. Hierzu gehören beispielsweise Vinylverbindungen, Allylverbindungen, Acrylverbindungen, Olefine, Diene, ungesättigte Halogenkohlenwasserstoffe bzw. deren entsprechend funktionalisierte Derivate.

Die erfindungsgemäßen Polymeren können beispielsweise mit Hilfe der Emulsionspolymerisation nach dem Stand der Technik, hergestellt werden. (Hans-Georg Elias, Makromoleküle, Hüthig & Wepf Verlag, Heidelberg, 1981, S. 603 ff.)

Weiterhin können zur Herstellung der erfindungsgemäßen Polymeren die Komponenten I, II und III auch in Lösung oder in Substanz nach den bekannten Verfahren copolymerisiert werden. (Hans-Georg Elias, Makromoleküle, Hüthig & Wepf Verlag, Heidelberg, 1981, S. 602 ff.)

Zur Copolymerisation der Komponenten I, II und III in Lösung können beispielsweise folgende Lösemittel eingesetzt werden:
Wasser, Aceton, Methylethylketon, Butanon, Cyclohexanon, Diethylether, Tetrahydrofuran, Dioxan, Methanol, Ethanol, Propanol, Butanol, Cyclohexanol, Dimethylacetamid, Dimethylsulfoxid, Dimethylformamid, Heptan, Cyclohexan, Benzol, Toluol, Dichlormethan, Trichlormethan, Ethylacetat, Propylacetat, Amylacetat und Acetonitril.

Als Polymerisationsinitiatoren lassen sich u. a. Azonitrile, Alkylperoxide, Acylperoxide, Hydroperoxide, Peroxoketone, Perester und Peroxocarbonate, Peroxodisulfat, Persulfat sowie alle üblichen Photoinitiatoren verwenden. Die Polymerisationinitiierung kann thermisch oder durch elektromagnetische Strahlung, wie z. B. UV-Licht oder γ-Strahlung erfolgen.

Werden zur Herstellung der erfindungsgemäßen Polymeren keine Carboxylat- und/oder Sulfonatgruppen-haltige Monomere eingesetzt, sondern deren funktionalisierte Derivate wie z. B. ein Carbonsäureester anstelle einer Carbonsäure, so müssen die funktionalisierten Derivate nach der Polymerisation in Carboxylat- oder Sulfonatgruppen überführt werden. Dies kann im Falle des Carbonsäureesters mittels basisch katalysierter Verseifung erfolgen. Die Derivatisierung von polymeren Materialien kann nach allgemein bekannten Verfahren (Hans Beyer, Lehrbuch der organischen Chemie, S. Hirzel Verlag, Stuttgart, 1988, S. 260 ff.) vorgenommen werden.

Erzeugnisse mit einer die Zellproliferation begünstigenden Oberfläche lassen sich direkt aus den erfindungsgemäßen Polymeren herstellen. Die erfindungsgemäßen Polymere können aber auch, gegebenenfalls als Lösungen in geeigneten Lösemitteln, durch Auftragetechniken wie Aufsprühen, Lackieren, Tauchen, Rakeln, Beschichten oder durch Mehrschichtenspritzgießen, Coextrusion bzw. Kalandrieren und Laminieren als dünne Schichten auf Standardpolymere aufgebracht werden.

Weiterhin ist eine Fixierung der erfindungsgemäßen Polymeren durch Primerschichten oder Zwischenschichten aus bifunktionellen Verbindungen auf gegebenenfalls aktivierten Standardpolymeren möglich.

Derartige Standardpolymere sind beispielsweise PVC, Polystyrol, Polyurethane, Polyacrylate, Polymethacrylate, Polyester, Polyether, Polyetherblockamide, Polyamide, Polycarbonate, Polyolefine, Silikone und Polytetrafluorethylen.

**Nachstehend ist das Meßverfahren zur Bestimmung der Zellproliferation beschrieben.**

### Herstellung der Zellsuspension

Menschliche Fibroblasten der Zelllinie ATCC CRL1696 (American Type Culture Collection, Rockville, Maryland, USA) werden in DMEM (Dulbecco's Modified Eagles Medium) unter Zusatz von Antibiotika, L-Glutamin und 10 % eines fötalen Kälberserums bei 37 °C unter einer Atmosphäre von 5 % CO₂ und 95 % Luft in Kulturflaschen gezüchtet. Nach Bebrütung wird das Nährmedium abgetrennt und der Zellrasen mit 0.05 % Trypsin/0,02 % EDTA für 5 Minuten behandelt. Anschließend werden die Zellen mit DMEM gewaschen und in demselben Nährmedium suspendiert.

### Messung der Zellproliferation

In einem 250 ml-Erlenmeyerkolben wird eine 2 x 2 cm große Polymerprobe auf eine Präpariernadel aufgespießt, mit Ethylenoxid sterilisiert und mit 20 ml des oben genannten Nährmediums versetzt. Dann wird die Polymerprobe mit 10⁵ Zellen aus der frisch hergestellten Zellsuspension beimpft und für 8 Tage bebrütet. Die Polymerprobe wird entnommen und mit steriler PBS-Pufferlösung gespült. Anschließend wird das Adenosintriphosphat aus den Zellen mit Hilfe von heißer Tris/EDTA-Lösung extrahiert und mit dem Bioluminiszenz Reagenz CLSII (Fa. Boehringer Mannheim GmbH, Mannheim) quantitativ bestimmt.

Als Referenzproben dienten auf die gleiche Weise vorbereitete Proben, die durch Polymerisation der Komponente III des jeweiligen erfindungsgemäßen Polymeren erhalten wurden. In einem Kontrollexperiment wurde eine Polymerprobe sofort nach dem Beimpfen mit der Zellsuspension gespült und die abgespülten Zellen nach der oben beschriebenen Methode quantitativ bestimmt. Die Begünstigung der Zellproliferation wird als prozentualer Quotient der ATP-Konzentration der auf den erfindungsgemäßen Polymeren gewachsenen Zellen dividiert durch den entsprechenden Wert der Referenzprobe ausgedrückt.

Die in den nachfolgenden Beispielen aufgeführten Meßergebnisse zeigen, daß die Zellproliferation auf erfindungsgemäßen Polymeren zwischen 60 % und 110 % zunimmt.

Die folgenden Beispiele sollen die Erfindung näher erläutern.

### Herstellung von Proben der erfindungsgemäßen Polymeren

### Beispiel 1.

In einer Stickstoffatmosphäre werden 223.2 g Methacrylsäuremethylester, 12.1 g Methacrylsäure und 4.9 g Natriumstyrolsulfonat in 500 ml Dimethylsulfoxid gelöst. Die Lösung wird unter Rühren auf 70° C erhitzt. Anschließend werden 2.3 g Azobisisobutyronitril gelöst in 30 ml Dimethylsulfoxid innerhalb von 2 Minuten zugetropft. Die Polymerisation wird über einen Zeitraum von 16 Stunden bei 70° C geführt. Im Anschluß wird das entstandene Produkt in einem vierfachen Überschuß Eiswasser gefällt, daraufhin 24 Stunden im Soxhlet mit Wasser extrahiert und bei 50° C im Vakuum getrocknet.

Eine anschließende Analyse der Zusammensetzung über ¹H-NMR ergibt:

| | |
|---|---|
| Methacrylsäure | 14 Mol-% |
| Natriumstyrolsulfonat | 4 Mol-% |
| Methacrylsäuremethylester | 82 Mol-% |

Aus diesen Werten ergibt sich ein Verhältnis der Carboxylatgruppen zu Sulfonatgruppen von 3.4.

### Beispiel 2:

In einer Stickstoffatmosphäre werden 201.6 g Methacrylsäuremethylester, 25.9 g Acrylsäure und 4.9 g Natriumstyrolsulfonat in 500 ml Dimethylsulfoxid gelöst. Die Lösung wird unter Rühren auf 70° C erhitzt. Anschließend werden 2 g Azobisisobutyronitril gelöst in 30 ml Dimethylsulfoxid innerhalb von 2 Minuten zugetropft. Die Polymerisation wird über einen Zeitraum von 16 Stunden bei 70° C geführt. Im Anschluß wird das entstandene Produkt in einem vierfachen Überschuß Eiswasser gefällt, daraufhin 24 Stunden im Soxhlet mit Wasser extrahiert und bei 50° C im Vakuum getrocknet.

Eine anschließende Analyse der Zusammensetzung über ¹H-NMR ergibt:

| | |
|---|---|
| Acrylsäure | 18 Mol-% |
| Natriumstyrolsulfonat | 5 Mol-% |
| Methacrylsäuremethylester | 77 Mol-% |

Aus diesen Werten ergibt sich ein Verhältnis der Carboxylatgruppen zu Sulfonatgruppen von 3.6.

### Beispiel 3:

In einer Stickstoffatmosphäre werden 244.0 g Styrol, 2.6 g Methacrylsäure und 4.9 g Natriumstyrolsulfonat in 500 ml Dimethylsulfoxid gelöst. Die Lösung wird unter Rühren auf 70° C erhitzt. Anschließend werden 2.3 g Azobisisobutyronitril gelöst in 30 ml Dimethylsulfoxid innerhalb von 2 Minuten zugetropft. Die Polymerisation wird über einen Zeitraum von 20 Stunden bei 70° C geführt. Im Anschluß wird das entstandene Produkt in einem vierfachen Überschuß Eiswasser gefällt, daraufhin 24 Stunden im Soxhlet mit Wasser extrahiert und bei 50° C im Vakuum getrocknet.

Eine anschließende Analyse der Zusammensetzung über ¹H-NMR ergibt:

| | |
|---|---|
| Methacrylsäure | 10 Mol-% |
| Natriumstyrolsulfonat | 3 Mol-% |
| Styrol | 87 Mol-% |

Aus diesen Werten ergibt sich ein Verhältnis der Carboxylatgruppen zu Sulfonatgruppen von 3.3.

### Beispiel 4:

In einer Stickstoffatmosphäre werden 225 g Styrol, 14.2 g Acrylsäure und 9.9 g Natriumstyrolsulfonat in 500 ml Dimethylsulfoxid gelöst. Die Lösung wird unter Rühren auf 70 ° C erhitzt. Anschließend werden 2.3 g Azobisisobutyronitril gelöst in 30 ml Dimethylsulfoxid innerhalb von 2 Minuten zugetropft. Die Polymerisation wird über einen Zeitraum von 20 Stunden bei 70° C geführt. Im Anschluß wird das entstandene Produkt in einem vierfachen Überschuß Eiswasser gefällt, daraufhin 24 Stunden im Soxhlet mit Wasser extrahiert und bei 50° C im Vakuum getrocknet.

Eine anschließende Analyse der Zusammensetzung über ¹H-NMR ergibt:

| | |
|---|---|
| Acrylsäure | 21 Mol-% |
| Natriumstyrolsulfonat | 5 Mol-% |
| Styrol | 74 Mol-% |

Aus diesen Werten ergibt sich ein Verhältnis der Carboxylatgruppen zu Sulfonatgruppen von 4.2.

### Beispiel 5:

In einer Stickstoffatmosphäre werden 316.3 g n-Butylmethacrylat, 12.5 g Methacrylsäure und 4.9 g Natriumstyrolsulfonat in 500 ml Dimethylsulfoxid gelöst. Die Lösung wird unter Rühren auf 70° C erhitzt. Anschließend werden 2.3 g Azobisisobutyronitril gelöst in 30 ml Dimethylsulfoxid innerhalb von 2 Minuten zugetropft. Die Polymerisation wird über einen Zeitraum von 20 Stunden bei 70° C geführt. Im Anschluß wird das entstandene Produkt in einem vierfachen Überschuß Eiswasser gefällt, daraufhin 24 Stunden im Soxhlet mit Wasser extrahiert und bei 50° C im Vakuum getrocknet.

Eine anschließende Analyse der Zusammensetzung über ¹H-NMR ergibt:

| | |
|---|---|
| Methacrylsäure | 16 Mol-% |
| Natriumstyrolsulfonat | 4 Mol-% |
| n-Butylmethacrylat | 81 Mol-% |

Aus diesen Werten ergibt sich ein Verhältnis der Carboxylatgruppen zu Sulfonatgruppen von 4.0.

### Beispiel 6:

In einer Stickstoffatmosphäre werden 317 g n-Butylmethacrylat, 11.2 g Acrylsäure und 2.5 g Natriumstyrolsulfonat in 500 ml Dimethylsulfoxid gelöst. Die Lösung wird unter Rühren auf 70° C erhitzt. Anschließend werden 2.3 g Azobisisobutyronitril gelöst in 30 ml Dimethylsulfoxid innerhalb von 2 Minuten zugetropft. Die Polymerisation wird über einen Zeitraum von 16 Stunden bei 70° C geführt. Im Anschluß wird das entstandene Produkt in einem vierfachen Überschuß Eiswasser gefällt, daraufhin 24 Stunden im Soxhlet mit Wasser extrahiert und bei 50° C im Vakuum getrocknet.

Eine anschließende Analyse der Zusammensetzung über ¹H-NMR ergibt:

| | |
|---|---|
| Acrylsäure | 9 Mol-% |
| Natriumstyrolsulfonat | 2 Mol-% |
| n-Butylmethacrylat | 89 Mol-% |

Aus diesen Werten ergibt sich ein Verhältnis der Carboxylatgruppen zu Sulfonatgruppen von 4.5.

### Herstellung der Membranen aus erfindungsgemäßen Polymeren

### Beispiel 7:

Es wird eine 5%ige Dimethylsulfoxidlösung der erfindungsgemäßen Polymeren gemäß den Beispielen 1, 2 und 5 hergestellt. Die Lösung wird in eine Petrischale gegossen und der Probe wird das Lösemittel hei 80° C unter vermindertem Druck entzogen. Anschließend wird die so hergestellte Membran in Stücke von jeweils 2 cm x 2 cm zerkleinert und 24 Stunden mit Wasser extrahiert. Vor den folgenden biologischen Untersuchungen werden die Membranstücke in einer Michaelis-Pufferlösung (pH = 7.33) drei mal jeweils drei Stunden gewaschen und bis zur weiteren Untersuchung bei - 4° C aufbewahrt.

### Herstellung der Beschichtungen aus erfindungsgemäßen Polymeren

### Beispiel 8:

Es wird eine 5%ige Methylethylketonlösung des erfindungsgemäßen Polymeren gemäß Beispiel 3 hergestellt. In diese Lösung wird eine 10 cm x 8 cm x 0.04 cm große Polyamidfolie für 10 Sekunden getaucht. Die Folie wird entnommen und 10 Stunden bei 50° C unter vermindertem Druck getrocknet. Anschließend wird die mit dem erfindungsgemäßen Polymeren beschichtete Folie in Stücke von jeweils 2 cm x 2 cm zerkleinert und 24 Stunden mit Wasser extrahiert. Vor den folgenden biologischen Untersuchungen werden die Proben in einer Michaelis-Pufferlösung (pH = 7.33) drei mal jeweils drei Stunden gewaschen und bis zur weiteren Untersuchung bei - 4° C aufbewahrt.

### Beispiel 9:

Es wird eine 5%ige Acetonlösung des erfindungsgemäßen Polymeren gemäß Beispiel 4 hergestellt. In diese Lösung wird eine 10 cm x 8 cm x 0.03 cm große Polyethylenfolie, deren Oberfläche zuvor durch 3 minütige Bestrahlung mit der 172 nm Strahlung eines Excimerstrahlers aktivierte wurde, 15 Sekunden eingetaucht. Die Folie wird entnommen und 10 Stunden bei 50° C unter vermindertem Druck getrocknet. Anschließend wird die beschichtete Folie in Stücke von jeweils 2 cm x 2 cm zerkleinert und 24 Stunden mit Wasser extrahiert. Vor den folgenden biologischen Untersuchungen werden die Proben in einer Michaelis-Pufferlösung (pH = 7.33) drei mal jeweils drei Stunden gewaschen und bis zur weiteren Untersuchung bei - 4° C aufbewahrt.

### Beispiel 10:

Es wird eine 5%ige Acetonlösung des erfindungsgemäßen Polymeren gemäß Beispiel 6 hergestellt. In diese Lösung wird eine 10 cm x 8 cm x 0.04 cm große Polyetherblockamidfolie für 10 Sekunden getaucht. Die Folie wird entnommen und 10 Stunden bei 50° C unter vermindertem Druck getrocknet. Anschließend wird die beschichtete Folie in Stücke von jeweils 2 cm x 2 cm zerkleinert und 24 Stunden mit Wasser extrahiert. Vor den folgenden biologischen Untersuchungen werden die Proben in einer Michaelis-Pufferlösung (pH = 7.33) drei mal jeweils drei Stunden gewaschen und bis zur weiteren Untersuchung bei - 4° C aufbewahrt.

### Konditionierung der Proben aus erfindungsgemäßen Polymeren

### Beispiel 11:

Die Membranen gemäß Beispiel 7 und die mit den erfindungsgemäßen Polymeren beschichteten Folien gemäß den Beispielen 8 bis 10 werden durch 15 minütige Bestrahlung mit ultraviolettem Licht sterilisiert. Anschließend werden die so vorbehandelten Proben drei mal jeweils drei Stunden in einer 0.15 molaren Natriumchloridlösung gehalten, darauf 3 Stunden mit destilliertem Wasser gewaschen. Im folgenden Reinigungsschritt werden sie drei mal jeweils drei Stunden in eine Phosphatpufferlösung folgender Zusammensetzung gelegt:

| | |
|---|---|
| CaCl₂ * H₂O | 0.132 g/l |
| KCl | 0.2 g/l |
| KH₂PO₄ | 0.2 g/l |
| MgCl₂ * 6H₂O | 0.1 g/l |
| NaCl | 8 g/l |
| Na₂HPO₄ | 1.15 g/l |

Im Anschluß daran werden die Proben erneut 15 Minuten mit ultraviolettem Licht bestrahlt. Die so vorhandenen Proben werden ca. 16 Stunden bei 37 °C in einer DMEM-Lösung (Dulbecco's Modified Eagles Medium) gelagert. Abschließend werden die Proben weitere 16 Stunden in einer mit Antibiotika, L-Glutamin und 10 Vol.-% eines fötalen Kälberserums versetzten DMEM-Lösung bei 37 °C und einer Atmosphäre von 5 % CO₂ und 95 % Luft gehalten.

Die nach den Beispielen 1, 2 und 5 hergestellten erfindungsgemäßen Polymeren wurden zu Membranen (Beispiel 7) verarbeitet. Auf Standardpolymere (Beispiele 8 bis 10) wurden erfindungsgemäße Polymere gemäß den Beispielen 3, 4 und 6 aufgebracht. Anschließend wurde eine Konditionierung dieser Proben nach Beispiel 11 durchgeführt und die Zellproliferation gemäß dem beschriebenen Verfahren bestimmt.

Die nachfolgende Tabelle zeigt die relative Besiedlung der erfindungsgemäßen Polymeren durch menschliche Fibroblasten.

| Erfindungsgemäßes Polymer gemäß Beispiel | Referenzpolymer | Relative Besiedlung in % (Referenzpolymer = 100) |
|---|---|---|
| 1 | Polymethylmethacrylat | 165 |
| 2 | Polymethylmethacrylat | 181 |
| 3 | Polystyrol | 161 |
| 4 | Polystyrol | 217 |
| 5 | Poly-n-Butylmethacrylat | 173 |
| 6 | Poly-n-Butylmethacrylat | 162 |
| Kontrollprobe, t = 0 h | Polystyrol | 1.8 |

## Patentansprüche

1. Verwendung von einem wasserunlöslichen, Carboxylat- und Sulfonat-gruppen enthaltenden Polymer, erhältlich durch radikalische Copolymerisation von:
- einem oder mehrerer Carboxylatgruppen-haltigen, aliphatisch ungesättigten Monomeren oder den entsprechend funktionalisierten Derivaten der Monomeren als Komponente I mit
- einem oder mehreren Sulfonatgruppen-haltigen, aliphatisch ungesättigten Monomeren oder den entsprechend funktionalisierten Derivaten der Monomeren als Komponente II und
- einer Komponente III, die ein aliphatisch ungesättigtes Monomer oder mehrere aliphatisch ungesättigte Monomere enthält,
wobei die entsprechend funktionalisierten Derivate nach der Copolymerisation in Carboxylat- und Sulfonatgruppen überführt werden, und wobei das Verhältnis der im Polymer enthaltenden Carboxylatgruppen zu Sulfonatgruppen 3 bis 10 ist,
zur der Begünstigung der Zellproliferation.

2. Verwendung nach Anspruch 1, **dadurch gekennzeichnet, daß** das Polymer enthält:
. eine Komponentel aus Acrylsäure oder Methacrylsäure gewählt,
. eine Komponente II dien Natriumstyrolsulfonat ist, und
. eine Komponente III aus Methacrylsäuremethylester, n-Butylmethacrylat und Styrol gewählt.

3. Verwendung nach Anspruch 1 oder 2, **dadurch gekennzeichnet, daß** die Summe der Anteile von Komponente I und Komponente II 5 bis 30 Mol-% des Polymeren beträgt.

4. Verwendung nach Anspruch 3, **dadurch gekennzeichnet, daß** die Summe der Anteile von Komponente I und Komponente II 5 bis 20 Mol-% des Polymeren beträgt.

5. Verwendung nach den Ansprüchen 1 bis 4, **dadurch gekennzeichnet, daß** das Verhältnis der im Polymeren enthaltenen Carboxylatgruppen zu Sulfonatgruppen 3 bis 5 ist.

6. Ein wasserunlösliches, Carboxylat- und Sulfonat-gruppen enthaltendes Polymer, **dadurch gekennzeichnet, daß** das Polymer enthält:
. eine Komponente I aus Acrylsäure oder Methacrylsäure gewählt,
. eine Komponente II dien Natriumstyrolsulfonat ist, und
. eine Komponente III aus Methacrylsäuremethylester, n-Butylmethacrylat und Styrol gewählt.

7. Das Polymer nach Anspruch 6, **dadurch gekennzeichnet, daß** die Summe der Anteile von Komponente I und Komponente II 5 bis 30 Mol-% des Polymeren beträgt.

8. Das Polymer nach Anspruch 7, **dadurch gekennzeichnet, daß** die Summe der Anteile von Komponente I und Komponente II 5 bis 20 Mol-% des Polymeren beträgt.

9. Das Polymer nach den Ansprüchen 6 bis 8, **dadurch gekennzeichnet, daß** das Verhältnis der im Polymeren enthaltenen Carboxylatgruppen zu Sulfonatgruppen 3 bis 10 ist.

10. Das Polymer nach Anspruch 9, **dadurch gekennzeichnet, daß** das Verhältnis der im Polymeren enthaltenen Carboxylatgruppen zu Sulfonatgruppen 3 bis 5 ist.

11. Verfahren zur Herstellung von einem wasserunlöslichen, Carhoxylat- und Sulfonat-gruppen enthaltenden Polymeren nach den Ansprüchen 6 bis 10, **dadurch gekennzeichnet, daß** das Polymer durch radikalische Copolymerisation von:
. einer Komponente I aus Acrylsäure oder Methacrylsäure gewählt,
. einer Komponente II dien Natriumstyrolsulfonat ist, und
. einer Komponente III aus Methacrylsäuremethylester, n-Butylmethacrylat und Styrol gewählt,
erhalten wird.

12. Verwendung der wasserunlöslichen, Carboxylat- und Sulfonat-gruppen enthaltenden Polymeren nach den Ansprüchen 6 bis 10 zur Herstellung von Erzeugnissen mit einer die Zellproliferation begünstigenden Oberfläche.

13. Verwendung der wasserunlöslichen, Carboxylat- und Sulfonatgruppen enthaltenden Polymeren nach den Ansprüchen 6 bis 10 zur Herstellung von Erzeugnissen mit einer die Zellproliferation begünstigende Beschichtung aus dem Polymeren.

14. Verwendung nach Anspruch 12 oder 13, dadurch gckennzeichnet, daß die Erzeugnisse medizintechnische Artikel sind.

15. Verwendung nach Anspruch 12 oder 13, **dadurch gekennzeichnet, daß** die Erzeugnisse künstliche Blutgefäße sind.

16. Erzeugnisse mit einer die Zellproliferation begünstigenden Oberfläche oder mit einer die Zellproliferation begünstigenden Beschichtung aus wasserunlöslichen, Carboxylat- und Sulfonat-gruppen enthaltenden Polymeren nach den Ansprüchen 6 bis 10.

17. Erzeugnisse nach Anspruch 16, **dadurch gekennzeichnet, daß** die Erzeugnisse medizintechnische Artikel sind.

18. Erzeugnisse nach Anspruch 17, **dadurch gekennzeichnet, daß** die mediziutechnische Artikel aus Kunststoffen, Keramiken oder Metallen sind.

19. Erzeugnisse nach Anspruch 16, **dadurch gekennzeichnet, daß** die Erzeugnisse künstliche Blutgefäße sind.

## Claims

1. Application of a water-insoluble polymer containing carboxylate and sulfonate groups, obtained by radical copolymerisation of
- one or more aliphatic unsaturated monomers containing carboxylate groups or the corresponding functionalised derivatives of the monomers as component I with
- one or more aliphatic unsaturated monomers containing sulfonate groups or the corresponding functionalised derivatives of the monomers as component II and
- a component III, that contains an aliphatic unsaturated monomer or several aliphatic unsaturated monomers,
wherein the corresponding functionalised derivatives following copolymerisation are reduced into carboxylate and sulfonate groups and wherein the ratio of the carboxylate groups to the sulfonate groups contained in the polymer is between 3 and 10, to promote proliferation of cells.

2. Application according to claim 1, **characterized in that** the polymer contains:
• a component I selected from either acrylic acid or methacrylic acid,
• a component II that is sodium styrene sulfonate, and
• a component III selected from methacrylic acid methyl ester, n-butyl methacrylate and styrene.

3. Application according to claim 1 or 2, **characterized in that** the total of the proportions of component I and component II are between 5 and 30 Mol-% of the polymer.

4. Application according to claim 3, **characterized in that** the total of the proportions of component I and component II is between 5 and 20 Mol-% of the polymer.

5. Application according to claims 1 to 4, **characterized in that** the ratio of the carboxylate groups to the sulfonate groups contained in the polymer is between 3 and 5.

6. A water-insoluble polymer containing carboxylate and sulfonate groups, **characterized in that** the polymer contains:
• a component I selected from either acrylic acid or methacrylic acid,
• a component II that is sodium styrene sulfonate , and
• a component III selected from methacrylic acid methyl ester, n-butyl methacrylate and styrene.

7. The polymer according to claim 6, **characterized in that** the total of the proportions of component I and component II is between 5 and 30 Mol-% of the polymer.

8. The polymer according to claim 7, **characterized in that** the total of the proportions of component I and component II is between 5 and 20 Mol-% of the polymer.

9. The polymer according to claims 6 to 8, **characterized in that** the ratio of the carboxylate groups to sulfonate groups contained in the polymer is between 3 and 10.

10. The polymer according to claim 9, **characterized in that** the ratio of the carboxylate groups to sulfonate groups contained in the polymer is between 3 and 5.

11. Method for manufacturing a water-insoluble polymer containing carboxylate and sulfonate groups according to claims 6 to 10, **characterized in that** the polymer is obtained by radical copolymerisation of:
• a component I selected from either acrylic acid or methacrylic acid,
• a component II that is sodium styrene sulfonate, and
• a component III selected from methacrylic acid methyl ester, n-butyl methacrylate and styrene.

12. Application of the water-insoluble polymer containing carboxylate and sulfonate groups according to claims 6 to 10 to the manufacture of products with a surface that promotes proliferation of cells.

13. Application of the water-insoluble polymer containing carboxylate and sulfonate groups according to claims 6 to 10 to the manufacture of products with a coating that promotes proliferation of cells made from the polymer.

14. Application according to claim 12 or 13, **characterized in that** the products are medicinal items.

15. Application according to claim 12 or 13, **characterized in that** the products are synthetic blood vessels.

16. Products with a surface that promotes proliferation of cells or with a coating that promotes proliferation of cells made from water-insoluble polymers containing carboxylate groups and sulfonate groups according to claims 6 to 10.

17. Products according to claim 16, **characterized in that** the products are medicinal items.

18. Products according to claim 17, **characterized in that** the medicinal items are made from synthetic materials, ceramics or metals.

19. Products according to claim 16, **characterized in that** the products are synthetic blood vessels.

## Revendications

1. Utilisation d'un polymère insoluble dans l'eau contenant des groupes carboxylate et sulfonate, qui peut être obtenu par copolymérisation radicalaire de :
- un ou plusieurs monomères aliphatiques insaturés contenant des groupes carboxylate, ou bien des dérivés fonctionnalisés de manière appropriée des monomères, en tant que composant I avec
- un ou plusieurs monomères aliphatiques insaturés contenant des groupes sulfonate, ou bien des dérivés fonctionnalisés de manière appropriée des monomères, en tant que composant II et
- un composant III contenant un monomère aliphatique insaturé ou plusieurs monomères aliphatiques insaturés,
les dérivés fonctionnalisés de manière appropriée étant convertis en groupes carboxylate et sulfonate après la copolymérisation et le ratio des groupes carboxylate sur les groupes sulfonate contenus dans le polymère allant de 3 à 10, afin de favoriser la prolifération cellulaire.

2. Utilisation selon la revendication 1, **caractérisée en ce que** le polymère contient :
- un composant I choisi parmi l'acide acrylique ou l'acide méthacrylique,
- un composant II qui est le styrènesulfonate de sodium, et
- un composant III choisi parmi le méthacrylate de méthyle, le méthacrylate de n-butyle et le styrène.

3. Utilisation selon la revendication 1 ou la revendication 2, **caractérisée en ce que** la somme des teneurs en composant I et en composant II représente de 5 à 30% molaires du polymère.

4. Utilisation selon la revendication 3, **caractérisée en ce que** la somme des teneurs en composant I et en composant II représente de 5 à 20% molaires du polymère.

5. Utilisation selon les revendications 1 à 4, **caractérisée en ce que** le ratio des groupes carboxylate sur les groupes sulfonate contenus dans le polymère va de 3 à 5.

6. Polymère insoluble dans l'eau contenant des groupes carboxylate et sulfonate, **caractérisé en ce que** le polymère contient :
- un composant I choisi parmi l'acide acrylique ou l'acide méthacrylique,
- un composant II qui est le styrènesulfonate de sodium, et
- un composant III choisi parmi le méthacrylate de méthyle, le méthacrylate de n-butyle et le styrène.

7. Polymère selon la revendication 6, **caractérisé en ce que** la somme des teneurs en composant I et en composant II représente de 5 à 30% molaires du polymère.

8. Polymère selon la revendication 7, **caractérisé en ce que** la somme des teneurs en composant I et en composant II représente de 5 à 20% molaires du polymère.

9. Polymère selon les revendications 6 à 8, **caractérisé en ce que** le ratio des groupes carboxylate sur les groupes sulfonate contenus dans le polymère va de 3 à 10.

10. Polymère selon la revendication 9, **caractérisé en ce que** le ratio des groupes carboxylate sur les groupes sulfonate contenus dans le polymère va de 3 à 5.

11. Procédé de fabrication d'un polymère insoluble dans l'eau contenant des groupes carboxylate et sulfonate selon les revendications 6 à 10, **caractérisé en ce que** le polymère est obtenu par copolymérisation radicalaire de :
- un composant I choisi parmi l'acide acrylique ou l'acide méthacrylique,
- un composant II qui est le styrènesulfonate de sodium, et
- un composant III choisi parmi le méthacrylate de méthyle, le méthacrylate de n-butyle et le styrène.

12. Utilisation du polymère insoluble dans l'eau contenant des groupes carboxylate et sulfonate selon les revendications 6 à 10 pour fabriquer des produits ayant une surface qui favorise la prolifération cellulaire.

13. Utilisation du polymère insoluble dans l'eau contenant des groupes carboxylate et sulfonate selon les revendications 6 à 10 pour fabriquer des produits ayant un revêtement, réalisé à partir du polymère, qui favorise la prolifération cellulaire.

14. Utilisation selon la revendication 12 ou la revendication 13, **caractérisée en ce que** les produits sont des articles médico-techniques.

15. Utilisation selon la revendication 12 ou la revendication 13, **caractérisée en ce que** les produits sont des vaisseaux sanguins artificiels.

16. Produits ayant une surface qui favorise la prolifération cellulaire ou un revêtement qui favorise la prolifération cellulaire réalisé(e) à partir de polymères insolubles dans l'eau contenant des groupes carboxylate et sulfonate selon les revendications 6 à 10.

17. Produits selon la revendication 16, **caractérisés en ce que** les produits sont des articles médico-techniques.

18. Produits selon la revendication 17, **caractérisés en ce que** les articles médico-techniques sont faits de matières plastiques, de céramiques ou de métaux.

19. Produits selon la revendication 16, **caractérisés en ce que** les produits sont des vaisseaux sanguins artificiels.
